# EUROPEAN PATENT APPLICATION

(11) **EP 1 923 001 A1**
(43) Date of publication of application: **21.05.2008**
(21) Application number: 07018334.8
(22) Date of filing: 19.09.2007
(51) Int. Cl.: A61B 8/00

(54) **Portable ultrasound device**

(30) Priority: 20.11.2006 KR 20060114652
(71) Applicant: MEDISON CO., LTD., Kangwon-do 250-870 (KR)
(72) Inventor: Kim, Jae, Gyoung, Seoul 135-280 (KR); Song, Young, Seuk, Seoul 135-280 (KR); Ahn, Mi, Jeoung, Seoul 135-280 (KR)
(74) Representative: Schmid, Wolfgang

(57) **Abstract**

Embodiments of the present invention may provide a portable ultrasound device comprising a probe, an image acquisition part, a processor and a communication module. The probe transmits an ultrasound signal to an object and receives the ultrasound signal reflected from the object. The image acquisition part acquires image of the object and forms a still image signal and a moving image signal. The processor receives the received ultrasound signal from the probe to form ultrasound image data, receives the still image signal and the moving image signal to form still image data and moving image data, and processes data transmitted from a medical facility. The communication module receives at least one of the ultrasound image data, still image data and moving image data from the processor to transmit them through an inter-network to the medical facility and receives the data transmitted from the medical facility to forward them to the processor.

## Description

The present application claims priority from Korean Patent Application No. 10-2006-114652 filed on November 20, 2006, the entire subject matter of which is incorporated herein by reference.

### BACKGROUND

### 1. Field

The present invention generally relates to ultrasound systems, and more particularly to a portable ultrasound device.

### 2. Background

An ultrasound system has become an important and popular diagnostic tool since it has a wide range of applications. Specifically, due to its non-invasive and nondestructive nature, the ultrasound system has been extensively used in the medical profession. Modern high-performance ultrasound systems and techniques are commonly used to produce two or three-dimensional diagnostic images of internal features of an object (e.g., human organs).

In order to transmit and receive ultrasound signals, the ultrasound system is generally provided with a probe including a wideband transducer. When the transducer is electrically stimulated, it produces ultrasound signals and transmits them into a human body. The ultrasound signals transmitted into the human body are reflected from borders between human tissues and then returned to the transducer. The returned ultrasound echo signals are converted into electric signals. Thereafter, ultrasound image data for imaging the tissues is produced by amplifying and signal-processing the echo signals.

However, such ultrasound systems are generally large in size and cannot be easily carried by hand. Thus, the conventional ultrasound systems can be used only in hospitals, ambulances and the like. As such, patients have no choice but to visit medical facilities equipped with ultrasound systems. Further, in case of emergencies (e.g., traffic accidents, heart attacks, etc.), it is difficult to accurately assess the patient in an ambulance and the like. Therefore, it is often difficult to provide emergency measures for the patients in such situations.

### BRIEF DESCRIPTION OF THE DRAWINGS

Arrangements and embodiments may be described in detail with reference to the following drawings in which like reference numerals refer to like elements and wherein:

FIG. 1 is a block diagram illustrating the configuration of a portable ultrasound device constructed in accordance with one embodiment of the present invention;

FIG. 2 is a diagram illustrating an example of a portable ultrasound device constructed in accordance with one embodiment of the present invention; and

FIG. 3 is a schematic diagram showing a portable ultrasound device interconnected to a medical facility through the Internet in accordance with one embodiment of the present invention.

### DETAILED DESCRIPTION OF THE PRESENT INVENTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

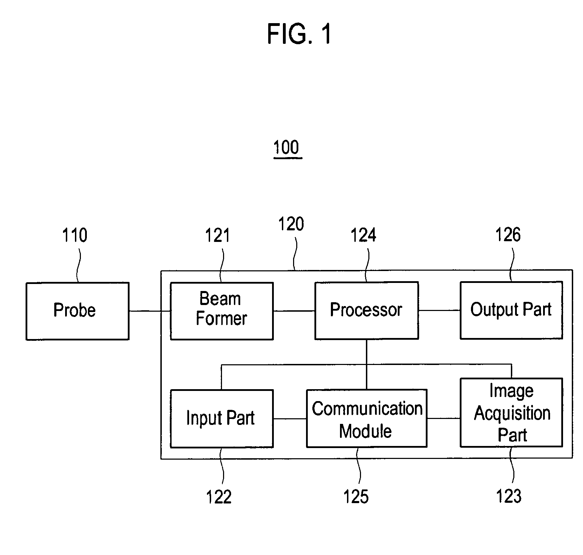

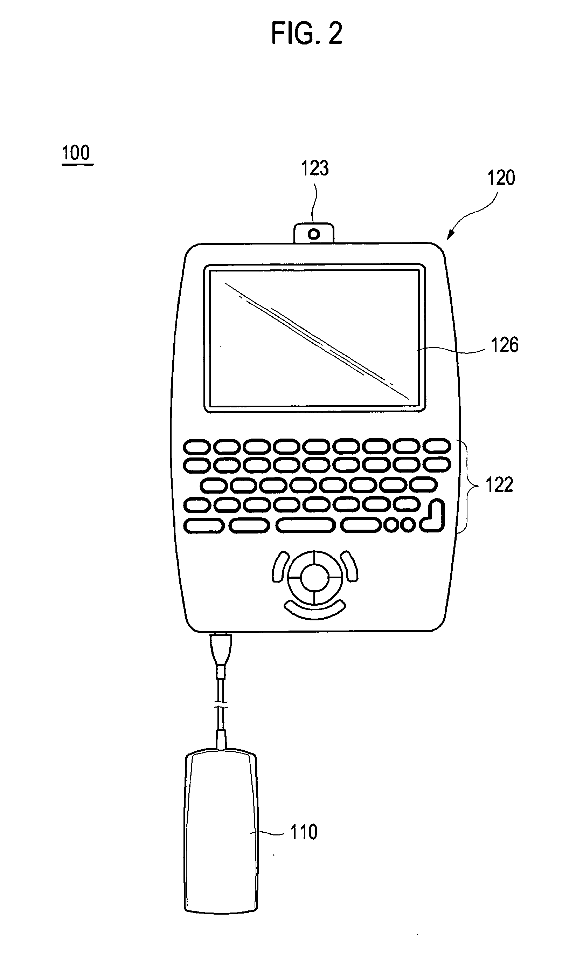

As illustrated in Figs. 1 and 2, an ultrasound device 100, which is constructed in accordance with one embodiment of the present invention, may include a probe 110 and a main body 120.

The probe 110 may include at least one transducer (not shown). The transducer may transmit an ultrasonic signal to an object in order to form an ultrasonic image of the object. Further, the probe 100 may receive the ultrasonic signal reflected from the object.

The main body 120 may include a beam former 121, an input part 122, an image acquisition part 123, a processor 124, a communication module 125 and an input part 126. Further, although not shown in the figures, the main body 120 may include a voice signal generating part (e.g., a microphone) for receiving a voice from an outside source and generating a voice signal. The main body 120 may also include a voice output part (e.g., a speaker) for receiving the voice signal and outputting the voice.

The beam former 121 may focus the ultrasonic signal, which is transmitted by at least one of the transducers of the probe 110, to the object. Further, the beam former 121 may also form an echo signal, which is formed by delaying and received focusing the ultrasound signal reflected from the object.

The input part 122 may receive a variety of information for manipulating the portable ultrasound device 100 from the user. Such information may include information on actuating and terminating the portable ultrasound device 100, selecting an image display mode (e.g., B-mode, C-mode, M-mode, Doppler mode, etc.), and initiating and terminating the formation of a still image signal and a moving image signal through the image acquisition part 123. Such information may further include information on accessing and terminating the access to a medical facility 300 through the Internet 230, initiating transmission of at least one of the still images and the moving images to the medical facility 300 and outputting data from the medical facility 300, etc. In one embodiment of the present invention, the input part 122 may comprise a keyboard, menu keys, a track ball, etc.

The image acquisition part 123 may acquire an image of the object (or the outward appearance of the object) and form an image signal(s) corresponding thereto. The image signal may be a still image signal or a moving image signal. In one embodiment of the present invention, the image acquisition part 123 may be installed at a prescribed location of the main body 120 in a fixed manner or in a manner such that the image acquisition part 123 can be freely attached and removed. Any device capable acquiring an image of an object and forming the image signal may be used as the image acquisition part 123. While the image acquisition part 123 was described as being installed in the main body 120, the image acquisition part 123 may be installed at a prescribed location of the portable ultrasound device 100 in a fixed manner or in a manner such that the image acquisition part 123 can be attached and removed.

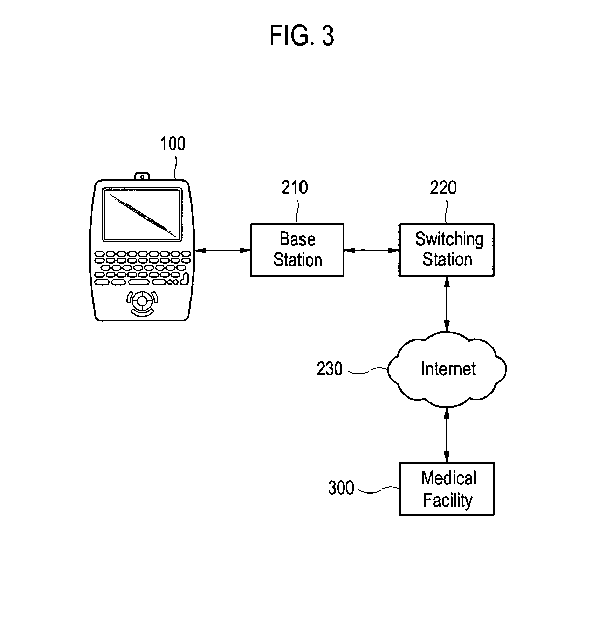

The processor 124 may generate ultrasound image data based on the echo signal from the beam former 121. The processor 124 may form still and/or moving image data based on the image signal (still image signal and/or moving image signal) from the image acquisition part 123. In one embodiment of the present invention, the processor 124 may further code the ultrasound, still and/or moving image data in accordance with a predetermined image coding algorithm. For example, the coded still image data may be in the JPEG (Joint Photographic Coding Experts Group), GIF (Graphics Interchange Format), TIFF (Tagged Image File Format) or any other similar still image coding format. Further, the coded moving image data may be in the MPEG (Moving Pictures Experts Group) or any other similar moving image coding format. The processor 124 may further transmit at least one of the still image data, the moving image data and the ultrasound image data through the communication module 125 to the medical facility 300 (shown in Fig. 3). It can also process data received from the medical facility 300 through the communication module 125. Further, the processor 124 may process the voice signal acquired from the voice signal acquisition part to form voice data, transmit the formed voice data to the medical facility 300 through the communication module 125, and process the voice data received through the communication module 125 from the medical facility 300 to output voice through the voice output part.

The communication module 125 may transmit data outputted from the processor 124 to a base station 210 (shown in Fig. 3) and receive data transmitted from the base station 210. The communication module 125 can be any device that can transmit and receive data. Here, the data may include at least one of the ultrasound image data, the still image data, the moving image data and the voice data.

The output part 126 may receive the ultrasound image data, the still image data and the moving image data processed by the processor 124 and output an ultrasound image, a still image and a moving image. The output part 126 can be any device that is compatible with the portable ultrasound diagnostic device 110. For example, the output part 126 may comprise a LCD (Liquid Crystal Display), a TFT (Thin Film Transistor) screen, a touch panel. etc.

As shown in Fig. 3, the base station 210 may receive data transmitted from the portable ultrasound device 100, transmit data transmitted from a switching center 220 to the portable ultrasound device 100, and transmit location information of the portable ultrasound device 100 to the switching station 220. The Internet 230 provides an access path that enables the portable ultrasound device 100 to access the medical facility 300 through the switching center 220 when the portable ultrasound device 100 accesses the Internet 230.

The medical facility 300 may receive at least one of still image data, moving image data and ultrasound image data transmitted from the portable ultrasound device 100, process the received data to form an image(s), and display the formed image at a display part (not shown). The medical facility 300 may receive the voice data transmitted from the portable ultrasound device 100, process the received voice data to form a voice signal, and output the formed voiced signal through a voice output part (e.g., speaker) (not shown). Further, the medical facility 300 may process a voice signal obtained from a voice acquisition part (e.g., microphone) (not shown) in order to form voice data and transmit the formed voice data to the portable ultrasound device 100. Further, the medical facility 300 may transmit data inputted by a user to the portable ultrasound device 100 through the Internet 230. In one embodiment of the present invention, the input data may include at least one of the result data determined by the user based on at least one of the still image, the moving image and the ultrasound image. The medical facility 300 may further include a storage part (not shown) for storing data on a plurality of still image and moving image, which are related to emergency measures. In addition, the medical facility 300 may transmit the above data on the still image and the moving image, which have been selected by the user.

As an example, one embodiment of the present invention was explained as installing a camera on a portable ultrasound device and obtaining at least one of a still image and a moving image by taking image of an object using the camera, and transmitting the obtained still image or the moving image and an ultrasound image to a medical facility. However, the present invention is not limited to the above. For example, the portable ultrasound device may recognize a digital camera and a mobile terminal (e.g., personal digital assistant, cellular phone, etc.) installed with a camera, receive still image data and moving image data of an object from the recognized digital camera and mobile terminal, and transmit the received still image data and the moving image data to a medical facility.

As mentioned above, according to one embodiment of the present invention, ultrasound image data and still/moving image data of an object may be transferred between a medical facility together with voice data. This enables faster and more accurate diagnosis of emergency patients and increases the chances of saving lives.

Also, according to one embodiment of the present invention, a user who knows nothing of medical emergency procedures may perform such procedures on an emergency patient based on a moving image provided from the medical facility.

In accordance with one embodiment of the present invention, there is provided a portable ultrasound device comprising a probe, an image acquisition part, a processor and a communication module. The probe transmits an ultrasound signal to an object and receives the ultrasound signal reflected from the object. The image acquisition part acquires an image of the object and forms a still image signal and a moving image signal. The processor receives the received ultrasound signal from the probe to form ultrasound image data, receives the still image signal and the moving image signal to form still image data and moving image data, and processes data transmitted from a medical facility. The communication module receives the formed data from the processor to transmit them through an inter-network to the medical facility and receives the data transmitted from the medical facility to forward them to the processor.

Any reference in this specification to "one embodiment," "an embodiment," "example embodiment," etc., means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to effect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. A portable ultrasound device interconnected with a medical facility through an inter-network, said device comprising:
a probe to transmit an ultrasound signal to an object and receive the ultrasound signal reflected from the object;
an image acquisition part to acquire an image of the object to form at least one of a still image signal and a moving image signal;
a processor to form ultrasound image data based on the received ultrasound signal from the probe, form at least one of still image data and moving image data based on at least one of the still image signal and the moving image signal from the image acquisition part, and processing data transmitted from the medical facility; and
a communication module to communicate at least one of the ultrasound image data, still image data and moving image data from the processor to the medical facility.

2. The device of Claim 1, further comprising:
a voice signal obtaining part to receive a first voice from an outside source and form a first voice signal based on the received voice; and
a voice output part to output a second voice based on a second voice signal from the processor.

3. The device of Claim 2, wherein said processor processes the first voice signal to form first voice data, transmits the formed first voice data through the communication module to the medical facility, processes second voice data transmitted from the medical facility to form the second voice signal, and outputs as voice the formed second voice signal through the voice output part.
